# EUROPEAN PATENT APPLICATION

(11) **EP 2 241 394 A1**
(43) Date of publication of application: **20.10.2010**
(21) Application number: 08867618.4
(22) Date of filing: 26.12.2008
(51) Int. Cl.: B22F 9/24, A61K 8/19, A61K 41/00, A61K 47/02, A61K 49/00, A61P 35/00, B22F 1/00, B22F 1/02, B22F 9/00, C09C 1/62, C12N 15/09

(54) **GOLD NANOPARTICLE COMPOSITION, DNA CHIP, NEAR INFRARED ABSORBENT, DRUG CARRIER FOR DRUG DELIVERY SYSTEM (DDS), COLORING AGENT, BIOSENSOR, COSMETIC, COMPOSITION FOR IN VIVO DIAGNOSIS AND COMPOSITION FOR THERAPEUTIC USE**

(30) Priority: 28.12.2007 JP 2007338733
(71) Applicant: Shiga University Of Medical Science, Otsu-shi Shiga 520-2121 (JP); I.S.T. Corporation, Otsu-shi Shiga 520-2153 (JP)
(72) Inventor: HOSOMI, Chisaka, Otsu-shi Shiga 520-2153 (JP); TOOYAMA, Ikuo, Otsu-shi Shiga 520-2121 (JP); INUBUSHI, Toshiro, Otsu-shi Shiga 520-2121 (JP); MORIKAWA, Shigehiro, Otsu-shi Shiga 520-2121 (JP); YAMADA, Hiroshi, Otsu-shi Shiga 520-2153 (JP)
(74) Representative: TBK-Patent
(86) International application number: PCT/JP2008/073847
(87) International publication number: WO 2009/084680

(57) **Abstract**

The objective of the present invention is to provide a gold nanoparticle composition that can pass through small holes *in vivo* more readily than a rod-shaped gold nanoparticle, and that can be utilized as a self-heating energy acceptor. The gold nanoparticle composition according to the present invention comprises a spherical gold nanoparticle and an organic ligand molecule. The organic ligand molecule bonds to the gold nanoparticle. Thus, the gold nanoparticle composition has at least one absorption peak wavelength (plasmon absorption wavelength) within the region of 600-1000 nm. Consequently, the gold nanoparticle composition self-heats when irradiated with electromagnetic waves having a wavelength of 600-1000 nm.

## Description

### TECHNICAL FIELD

The present invention relates to gold nanoparticle compositions that offer utility as, for example, biosensors, DNA chips, and diagnostic agents and therapeutic agents such as for tumors and the like.

### BACKGROUND ART

Currently, gold nanoparticles are utilized widely in industrial fields such as medical products, cosmetic products, food products, wiring materials, polarized materials, electrode materials, near infrared absorbent materials, forgery-proof inks, electromagnetic wave shielding materials, surface enhanced fluorescence sensors, biomarkers, nano-waveguides, recording materials, recording elements, drug carriers for drug delivery systems (DDSs), biosensors, DNA chips, test agents and the like (for example, see Patent Documents 1 and 2.)

It is known that, when the gold nanoparticles are irradiated with electromagnetic waves, the light absorption phenomenon referred to as plasmon absorption gives rise to self-heating. Furthermore, gold nanoparticles that differ in shape and size also differ in the absorption wavelengths. For example, gold nanoparticles generally have an absorption region in the vicinity of 530 nm, but rod-shaped gold nanoparticles with an aspect ratio of 1.1-8.0 have a long wavelength absorption region (400-1200 nm) caused by the long axis of the rod in addition to the absorption region in the vicinity of 530 nm caused by the short axis of the rod (for example, see Patent Documents 1 and 2).

Moreover, the gold nanoparticles are attracting attention in the medical field due to the low human toxicity, and particularly in the field of the diagnosis and treatment of tumors and the like. In fact, malignant tumors (i.e. cancer cells), for example, are known to undergo irreversible damage when they are heated to 40-50 °C. Thus, it has been suggested that, after an energy acceptor such as a gold nanoparticle is transported to a malignant tumor or the like *in vivo,* and the energy acceptor is supplied with energy from outside the body that causes the energy acceptor to generate heat, the malignant tumor or the like will be destroyed or inactivated (for example, see Patent Document 3).
Patent Document 1: Japanese Published Unexamined Patent Application No. 2005-320616
Patent Document 2: Japanese Published Unexamined Patent Application No. 2005-255582
Patent Document 3: Japanese Published Unexamined Patent Application No. 2007-521109

### INVENTION DISCLOSURE

### PROBLEM TO BE SOLVED BY THE INVENTION

Living organisms generally absorb electromagnetic waves with a wavelength ≤600 nm (ultraviolet and visible light region) or ≥1000 nm (infrared region). Specifically, the former electromagnetic waves are absorbed chiefly by pigments, hemoglobin or the like in the body, while the latter electromagnetic waves are absorbed chiefly by the water content inside the body. Consequently, even though gold nanoparticle compositions have the property that they absorb electromagnetic waves with a wavelength ≤600 nm or ≥1000 nm and generate heat, the gold nanoparticle compositions do not generate heat *in vivo.*

In addition, rod-shaped gold nanoparticles such as those mentioned above have plasmon absorption wavelengths in the region 550-1200 nm, and when irradiated from the exterior with such electromagnetic waves, the rod-shaped gold nanoparticles can self-heat inside the body. However, if it is necessary to transport the gold nanoparticles to an area of pathology through extremely small holes inside the body, for example if it is necessary to transport the gold nanoparticles to malignant tumors through holes of approximately 100 nm formed at the connection branch points between the pre-existing blood vessels and the new blood vessels produced by those malignant tumors, the rod-shaped gold nanoparticles will encounter difficulties reaching the area of pathology due to the shape.

The objective of the present invention is to provide a gold nanoparticle composition that can pass through small holes *in vivo* more readily than a rod-shaped gold nanoparticle, and that can be utilized as a self-heating energy acceptor.

### MEANS TO SOLVE THE PROBLEM

To overcome the above-stated problem, the present inventors used the results from carrying out many experiments related to the synthesis of gold nanoparticles, selection of organic ligand molecules, absorption wavelengths of gold nanoparticle compositions, heat generation properties and the like, at the completion of which achieved was a gold nanoparticle composition that has at least one absorption peak (plasmon absorption peak) in the wavelength range of 700-800 nm.

The gold nanoparticle composition according to the present invention comprises a spherical gold nanoparticle and an organic ligand molecule. The organic ligand molecule bonds to the gold nanoparticle. What is referred to as a "bond" is, for example, a coordination bond, a hydrogen bond, or the like. Thus, the gold nanoparticle composition has at least one absorption peak (plasmon absorption peak) wavelength within the region of 600-1000 nm. Moreover, it is more preferable to have at least one absorption peak wavelength within the region of 650-900 nm. There is little *in vivo* absorption of the electromagnetic waves in the wavelength region, and when a gold nanoparticle composition is present inside the body, energy acceptors efficiently absorb the electromagnetic waves of the wavelength region and can generate heat.

In addition, in the present invention, the gold nanoparticle is preferably manufactured by reduction of chlorauric acid in a chlorauric acid solution. Furthermore, examples of the methods for reduction of chlorauric acid in a chlorauric acid solution that can be named include the method of adding a reducing agent, the method of irradiating with ultraviolet rays, the method of adding an alcohol, the method of irradiating with ultrasonic waves and the like.

Moreover, in the present invention, the organic ligand molecule is an organic compound that has at least one functional group that bonds with the gold nanoparticle. For the functional group, sulfur-containing functional groups, nitrogen-containing functional groups, phosphorus-containing functional groups and oxygen-containing functional groups are preferred. Furthermore, the organic ligand molecule plays a role not only in determining the nano-size of the gold particle by suppressing the growth of the gold nanoparticle, but also in modifying the plasmon absorption wavelength of the gold particle composition.

In addition, in the present invention, organic ligand molecule that is at least one type of diamine as shown in generic formula (I) below are preferred.

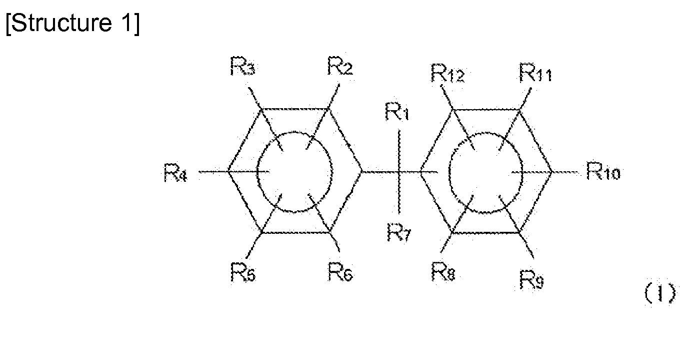

(where in the formula, R₁ and R₇ are substituents or linking groups selected from the group consisting of CH₃, CF₃, OH, H, COOH, halogen elements, phenyl group and acene-type aromatic hydrocarbons. R₂-R₆ are substituents or linking groups selected from the group consisting of H, OH, NH₂, SH, CH₃, halogen elements and COOH, and at least one from among R₂-R₆ is NH₂. In addition, R₈-R₁₂ are substituents or linking groups selected from the group consisting of H, OH, NH₂, SH, CH₃, halogen elements and COOH, and at least one from among R₈-R₁₂ is NH₂.)

Furthermore, among such diamines, the ones that are at least one type of diamine as shown in generic formula (II) below are preferred.

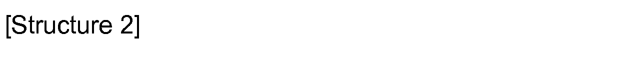

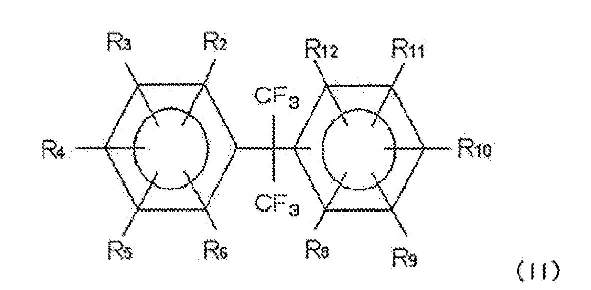

(where in the formula, R₂-R₆ are substituents or linking groups selected from the group consisting of H, OH, NH₂, SH, CH₃, halogen elements and COOH, and at least one from among R₂-R₆ is NH₂. In addition, R₈-R₁₂ are substituents or linking groups selected from the group consisting of H, OH, NH₂, SH, CH₃, halogen elements and COOH, and at least one from among R₈-R₁₂ is NH₂.)

In addition, among such diamines, 2,2-bis(3-aminophenyl)hexafluoropropane, 2,2-bis(3-amino-4-hydroxyphenyl)hexafluoropropane and 2,2-bis(4-aminophenyl)hexafluoropropane) as shown in the chemical structure formulas (III)-(V) below are particularly preferred. Furthermore, the diamines can be used either singly or in mixtures.

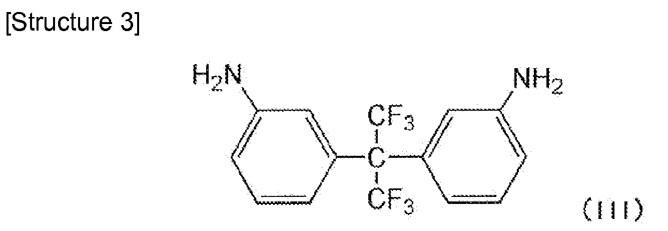

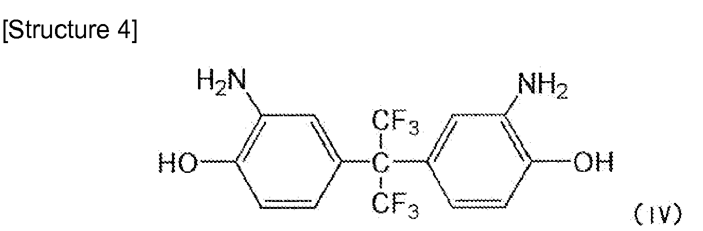

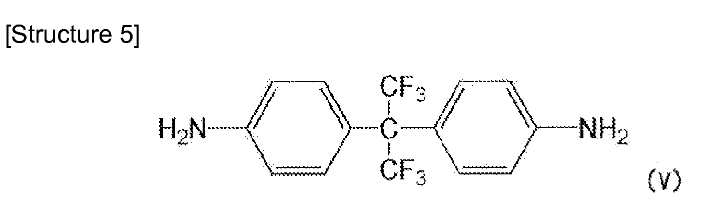

Furthermore, gold nanoparticle composition according to the present invention can be utilized in DNA chips, near infrared absorbent materials, drug carriers for drug delivery systems (DDSs), coloring agents, biosensors, cosmetic products, compositions for *in vivo* diagnosis, compositions for therapeutic use, and the like. Moreover, the gold nanoparticle composition can be used in the form of aqueous dispersions. In addition, the gold nanoparticle composition can be used in various applications such as coating agents, filling materials and the like.

Additionally, If a gold nanoparticle composition according to the present invention is introduced into the body using a nanoprobe and then is irradiated with electromagnetic waves having a wavelength ≥600 nm and ≤1000 nm, the nanoprobe will be heated. Consequently, a gold nanoparticle composition according to the present invention can be used for *in vivo* diagnosis and therapy. Such *in vivo* diagnosis and therapy is preferable because it is not necessary to cut open a person's body and the psychological and physical stress on the patient is slight.

### EFFECT OF THE INVENTION

At least one absorption peak wavelength of a gold nanoparticle composition according to the present invention is in the region 600-1000 nm where there is no absorption by the body. Consequently, the gold nanoparticle composition according to the present invention will self-heat even inside the body when irradiated from the exterior with electromagnetic waves with a wavelength in the region 600-1000 nm. Consequently, the gold nanoparticle composition can be utilized for hyperthermic treatment against cancer such as in an malignant tumor. Moreover, a gold nanoparticle composition according to the present invention is a spherical particle that are approximately 1 s to 10s of nm. Consequently, gold nanoparticle composition according to the present invention can pass through small holes *in vivo* more readily than a rod-shaped gold nanoparticle, for example, and it is possible for the gold nanoparticle composition to be introduced into minute locations such as the blood-brain barrier (BBB) in the brain and the like so that the gold nanoparticle composition uses are found in the diagnosis and therapy of brain tumors and the like.

For this reason, a gold nanoparticle composition according to the present invention can pass through small holes *in vivo* more readily than a rod-shaped gold nanoparticle and can be utilized as a self-heating energy acceptor.

Moreover, in addition to the above-mentioned applications, a gold nanoparticle composition according to the present invention can also be utilized as substrate materials such as in DNA chips, near infrared absorbent materials, drug carriers for drug delivery systems (DDSs), coloring agents, biosensors, cosmetic products, compositions for *in vivo* diagnosis, compositions for therapeutic use, extracorporeal diagnostic agents, test agents, biomarkers, wiring materials, electrode materials, catalysts, surface enhanced fluorescence sensors, surface enhanced Raman sensors, near infrared light-cut films, near infrared light-cut filters, near infrared light-cut glass, color filters, heat ray-cut filters, optical filter materials, wavelength absorbent materials, electromagnetic wave shielding materials, coating materials, coating films, electromagnetic wave shields, conductive pastes, conductive coating materials, conductive coating films, conductive films, nano-waveguides, forgery-proof inks, recording materials, recording elements and the like.

### BRIEF EXPLANATION OF DIAGRAMS

Figure 1 is a transmission-type electron photomicrograph of the spherical gold nanoparticle composition prepared in Working Example 1.
Figure 2 is an absorption spectrum of the spherical gold nanoparticle composition prepared in Working Example 1.
Figure 3 is a graph that shows the temperature-increase characteristics due to near infrared irradiation of the spherical gold nanoparticle composition prepared in Working Example 1.
Figure 4 is a schematic diagram of the measuring apparatus for measuring the temperature-increase characteristics due to near infrared irradiation of a spherical gold nanoparticle composition.
Figure 5 is an absorption spectrum of the spherical gold nanoparticle composition prepared in Working Example 2.
Figure 6 is an absorption spectrum of the spherical gold nanoparticle composition prepared in Working Example 3.
Figure 7 is an absorption spectrum of the spherical gold nanoparticle composition prepared in Working Example 4.

### EXPLANATION OF SYMBOLS

- 1: Agar gel
- 2: Cell
- 3: Near infrared filter
- 4: Light guide tube
- 5: Light power source
- 6: Glass fiber probe
- 7: Temperature measuring instrument

### PREFFERED EMBODIMENTS OF THE PRESENT INVENTION

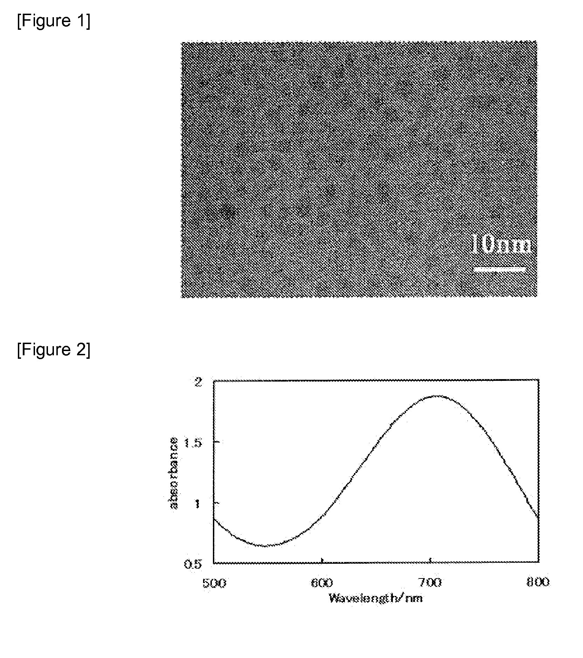

Spherical gold nanoparticle composition according to embodiments of the present invention is explained below. A gold nanoparticle of the spherical gold nanoparticle composition according to the present embodiment exhibits a true spherical shape of ≤10 nm as shown in Figure 1. Furthermore, "Spherical shape" in the present application means the approximate shape that can be identified as a true circle in the transmission-type electron photomicrograph magnification of Figure 1.

Moreover, Spherical gold nanoparticle composition according to the present embodiment will have at least one absorption peak wavelength within the region of 600-1000 nm. In other words, there is currently no gold nanoparticle composition that has a plasmon absorption wavelength within the region.

Furthermore, Plasmon absorption in the vicinity of 530 nm is generally produced in gold nanoparticles, but the plasmon absorption is shifted to longer wavelengths in the spherical gold nanoparticle composition according to the present invention. As far as can be predicted, this is supposed to arise from the structure of the organic ligand molecule that modifies the gold nanoparticle, or to the nature of the bond between the organic ligand molecule and the gold nanoparticle, because the plasmon absorption wavelength of the simple spherical gold nanoparticle alone (without the organic ligand molecule) is 530 nm, and no light absorption is observed on the long wavelength side of ≥600 nm for the simple 2,2-bis(3-aminophenyl)hexafluoropropane, 2,2-bis(3-amino-4-hydroxyphenyl)hexafluoropropane or 2,2-bis(4-aminophenyl)hexafluoropropane) alone.

In addition, for the spherical gold nanoparticle composition according to the present embodiment, depending on the requirements, functionalized molecules such as fluorescent dyes, hydrophilic polymers to increase the biocompatibility, dispersing agents, storage stabilizers, surfactants and the like can be added. Furthermore, examples of fluorescent dyes that can be named include fluoresceins (FITC), phycoerythrin, rhodamines and the like. Furthermore, examples of hydrophilic polymers that can be named include poly(ethylene glycol)s, taurine, poly(glutamic acid), poly(vinyl alcohol), polyvinylpyrrolidine, poly(acrylic acid)s, poly(amino acid)s and the like.

Spherical gold nanoparticle composition according to the present embodiment is further explained in detail in the working examples below.

### Working Example 1

To 0.2 mmol of an aqueous chlorauric acid solution (Wako Pure Chemical) was added 10 mL of ethanol, then 5 mL of ethanol in which was dissolved 0.5 mmol of 2,2-bis(3-aminophenyl)hexafluoropropane (Tokyo Kasei, referred to below as "33-6FD") was added, after which the mixture was stirred at room temperature for 5 min. Thereafter, the mixture was irradiated with ultraviolet light having a wavelength of 254 nm to obtain a dispersion of a spherical gold nanoparticle composition with 33-6FD as the organic ligand molecule.

Then, the dispersion of the spherical gold nanoparticle composition was dried to obtain the spherical gold nanoparticle composition, the particle size of which was measured by transmission-type electron microscopy (TEM, Hitachi model H7100TE). As shown in the transmission-type electron photomicrograph of Figure 1, the maximum particle diameter of the spherical gold nanoparticle composition was clarified as approximately 5-7 nm. Furthermore, the gold nanoparticle compositions are photographed as a plurality of black particles in the transmission-type electron photomicrograph of Figure 1.

Next, the plasmon absorption wavelength of the spherical gold nanoparticle composition was measured using a spectrophotometer (Amersham Biosciences model Ultraspec 2100). The absorption spectrum of the spherical gold nanoparticle composition of the present working example is shown in Figure 2. As is clear from Figure 2, the plasmon absorption wavelength of the spherical gold nanoparticle composition of the present working example was 720 nm.

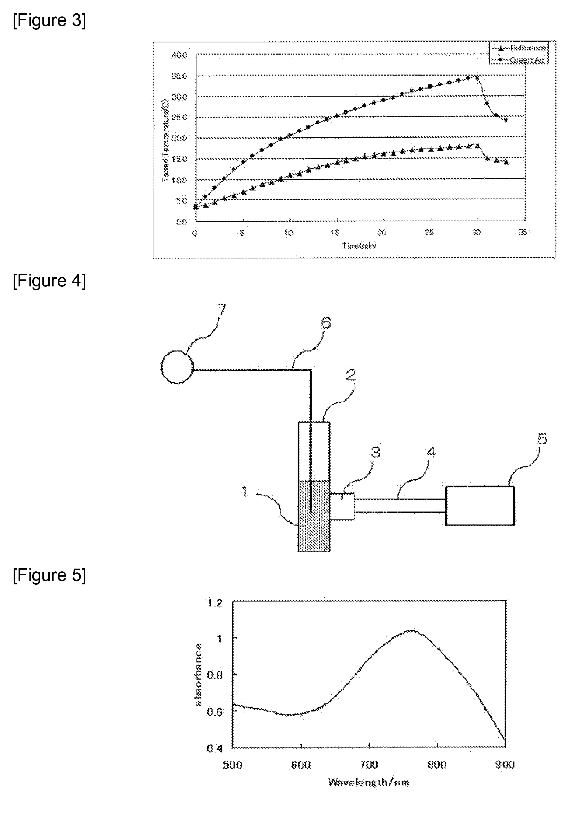

In addition, the temperature-increase characteristics of the spherical gold nanoparticle composition were determined using the measuring apparatus shown in Figure 4. The measurement conditions are as follows. First, agar gel 1 was placed in glass cell 2, and into the agar gel 1 was injected 10 mg of the spherical gold nanoparticle composition. Next, following insertion of glass fiber probe 6 into the agar gel 1, the temperature of agar gel 1 was measured with glass fiber probe 6 subjecting same to near infrared radiation of 800-1050 nm. Furthermore, the temperature measurement was carried out for 30 minutes after the time when the near infrared irradiation began.

In addition, in Figure 4, 3 signifies a near infrared filter, 4 signifies a light guide tube, 5 signifies a light power source and 7 signifies a temperature measuring instrument.

Thus, the results of the measurement clarified that the spherical gold nanoparticle composition of the present working example raised the temperature to 34 °C after 30 minutes of near infrared irradiation. Moreover, the temperature from the heat generation was approximately double that of the reference (agar gel without the spherical gold nanoparticle composition).

### Working Example 2

Except for substituting 2,2-bis(3-amino-4-hydroxyphenyl)hexafluoropropane (Tokyo Kasei, referred to below as "33-6HFD") for 33-6FD, the working example 2 was carried out in the same manner as for Working Example 1 to yield a spherical gold nanoparticle composition dispersion with 33-6HFD as the organic ligand molecule. Moreover, the measurements of the plasmon absorption wavelength and temperature-increase characteristics were determined for the spherical gold nanoparticle composition in the same manner as for Working Example 1.

The absorption spectrum of the spherical gold nanoparticle composition of the present working example is shown in Figure 5. It is clear from Figure 5 that the plasmon absorption wavelength for the spherical gold nanoparticle composition of the present working example is 760 nm.

Moreover, it was clarified that the spherical gold nanoparticle composition of the present working example raised the temperature to 33 °C after 30 minutes of near infrared irradiation.

### Working Example 3

Except for substituting 2.0 mmol of 2,2-bis(4-aminophenyl)hexafluoropropane (Tokyo Kasei, referred to below as "44-6FD") for 0.5 mmol of 33-6FD, the working example 3 was carried out in the same manner as for Working Example 1 to yield a spherical gold nanoparticle composition dispersion with 44-6FD as the organic ligand molecule. Moreover, the measurement of the plasmon absorption wavelength was carried out for the spherical gold nanoparticle composition in the same manner as for Working Example 1.

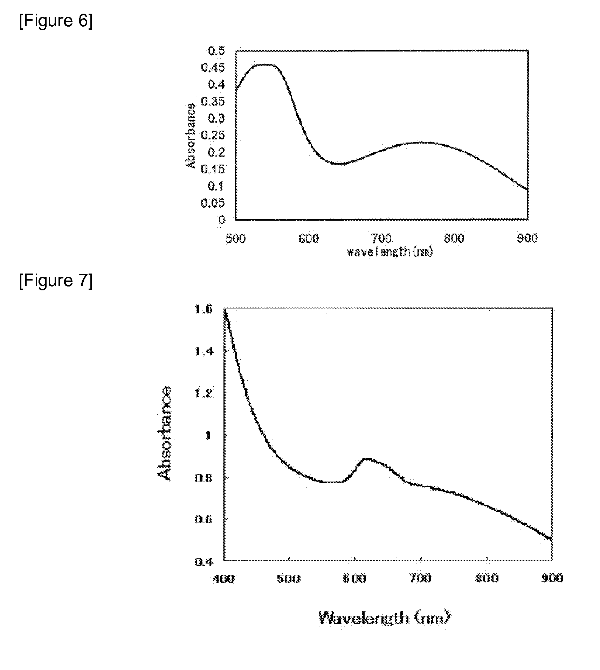

The absorption spectrum of the spherical gold nanoparticle composition of the present working example is shown in Figure 6. It is clear from Figure 6 that the spherical gold nanoparticle composition of the present working example exhibits a plasmon absorption wavelength of 550 nm together with a second plasmon absorption peak at 760 nm.

### Working Example 4

To 50 mg of the dispersion of the spherical gold nanoparticle composition prepared in Working Example 1 was added 10 mL of ethanol followed by addition of 5 mL of water in which was dissolved 0.1 mmol of taurine (Tokyo Kasei), after which the mixture was stirred at room temperature for 48 hours. Moreover, the measurement of the plasmon absorption wavelength of the spherical gold nanoparticle composition was carried out in the same manner as for Working Example 1.

The absorption spectrum of the spherical gold nanoparticle composition of the present working example is shown in Figure 7. It is clear from Figure 7 that the plasmon absorption wavelength of the spherical gold nanoparticle composition of the present working example is 620 nm.

### Industrial applicability

The spherical gold nanoparticle composition according to the present invention has substantial plasmon absorption peaks in the vicinity of 620 nm, 720 nm, and 760 nm, generates heat when subjected to near infrared radiation (600-900 nm), and is thereby useful for the diagnosis and hyperthermic treatment of cancer cells.
Moreover, the spherical gold nanoparticle composition according to the present invention can be suitably utilized in DNA chips, near infrared absorbent materials, drug carriers for drug delivery systems (DDSs), coloring agents, biosensors, cosmetic products, compositions for *in vivo* diagnosis, compositions for therapeutic use, extracorporeal diagnostic agents, test agents, biomarkers, wiring materials, electrode materials, catalysts, surface enhanced fluorescence sensors, surface enhanced Raman sensors, near infrared light-cut films, near infrared light-cut filters, near infrared light-cut glass, color filters, heat ray-cut filters, optical filter materials, wavelength absorbent materials, electromagnetic wave shielding materials, coating materials, coating films, electromagnetic wave shields, conductive pastes, conductive coating materials, conductive coating films, conductive films, nano-waveguides, forgery-proof inks, recording materials, recording elements and the like.

## Claims

1. A gold nanoparticle composition
that comprises
a spherical gold nanoparticle and
an organic ligand molecule that are bonded to the gold nanoparticle and that has at least one absorption peak wavelength within the region of 600-1000 nm.

2. The gold nanoparticle composition as recited in Claim 1, wherein the gold nanoparticle is manufactured by the reduction of chlorauric acid in a chlorauric acid solution.

3. The gold nanoparticle composition as recited in Claims 1 or 2, wherein the organic ligand molecule includes at least one atom from among nitrogen atom and sulfur atom.

4. The gold nanoparticle composition as recited in Claim 3, wherein the organic ligand molecule includes at least one type of diamine as shown in the generic formula (I) below. 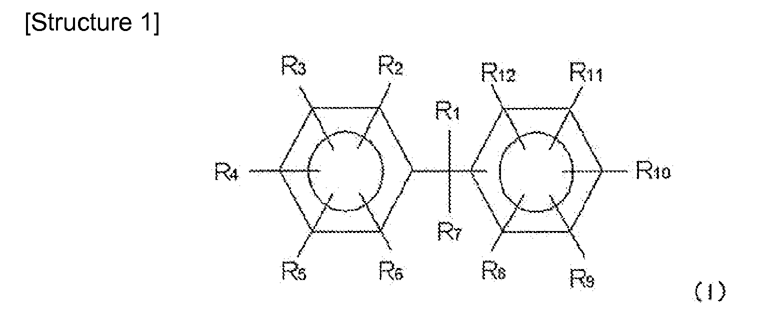 (where in the formula, R₁ and R₇ are substituents or linking groups selected from the group consisting of CH₃, CF₃, OH, H, COOH, halogen elements, phenyl group and acene-type aromatic hydrocarbons. R₂-R₆ are substituents or linking groups selected from the group consisting of H, OH, NH₂, SH, CH₃, halogen elements and COOH, and at least one from among R₂-R₆ is NH₂. In addition, R₈-R₁₂ are substituents or linking groups selected from the group consisting of H, OH, NH₂, SH, CH₃, halogen elements and COOH, and at least one from among R₈-R₁₂ is NH₂.)

5. The gold nanoparticle composition as recited in Claim 3, wherein the organic ligand molecule includes at least one type of diamine as shown in the generic formula (II) below. 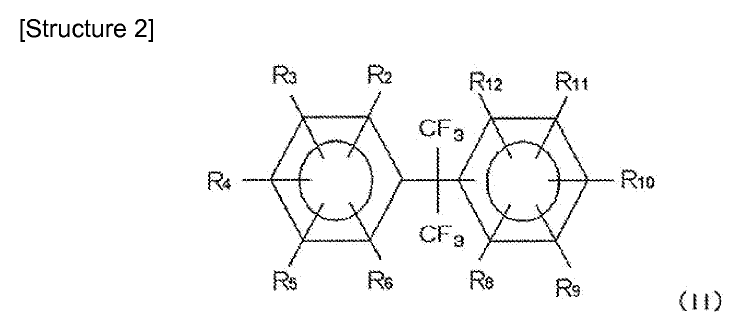 (where in the formula, R₂-R₆ are substituents or linking groups selected from the group consisting of H, OH, NH₂, SH, CH₃, halogen elements and COOH, and at least one from among R₂-R₆ is NH₂. In addition, R₈-R₁₂ are substituents or linking groups selected from the group consisting of H, OH, NH₂, SH, CH₃, halogen elements and COOH, and at least one from among R₈-R₁₂ is NH₂.)

6. The gold nanoparticle composition as recited in Claim 3, wherein the organic ligand molecule is at least one diamine selected from the group consisting of the diamine shown in chemical structure (III) below, the diamine shown in chemical structure (IV) below and the diamine shown in chemical structure (V) below.

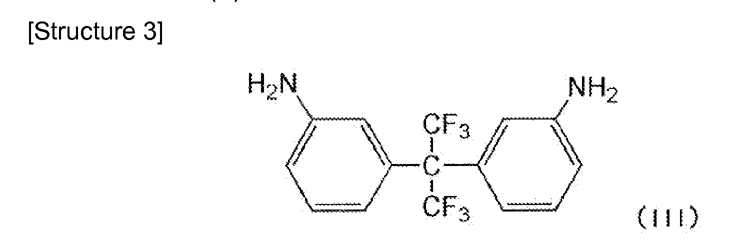

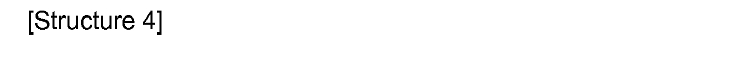

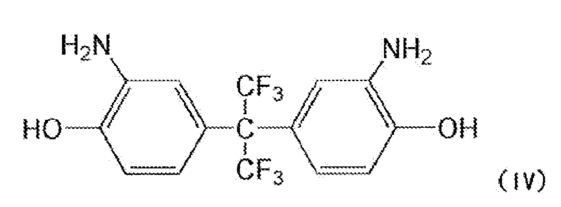

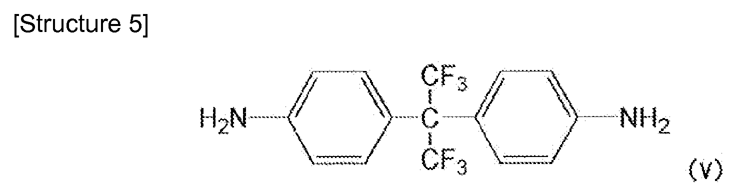

7. A DNA chip comprising a gold nanoparticle composition as recited in any of Claims 1 through 6.

8. A near infrared absorbent material comprising a gold nanoparticle composition as recited in any of Claims 1 through 6.

9. A drug carrier for a drug delivery system comprising a gold nanoparticle composition as recited in any of Claims 1 through 6.

10. A coloring agent comprising a gold nanoparticle composition as recited in any of Claims 1 through 6.

11. A biosensor comprising a gold nanoparticle composition as recited in any of Claims 1 through 6.

12. A cosmetic product comprising a gold nanoparticle composition as recited in any of Claims 1 through 6.

13. A composition for *in vivo* diagnosis comprising a gold nanoparticle composition as recited in any of Claims 1 through 6.

14. A composition for therapeutic use comprising a gold nanoparticle composition as recited in any of Claims 1 through 6.
